# EUROPEAN PATENT APPLICATION

(11) **EP 2 281 884 A1**
(43) Date of publication of application: **09.02.2011**
(21) Application number: 09731254.0
(22) Date of filing: 24.02.2009
(51) Int. Cl.: C12N 15/09, A61K 35/76, A61K 48/00, A61P 35/00

(54) **MESOTHELIOMA-SPECIFIC PROMOTER AND USE THEREOF**

(30) Priority: 11.04.2008 JP 2008104070
(71) Applicant: National University Corporation Okayama University, Kita-ku Okayama-shi Okayama 700-8530 (JP)
(72) Inventor: TANAKA, Noriaki, Kita-ku, Okayama-shi Okayama 700-8558 (JP); MATSUOKA, Junji, Okayama-shi Okayama 700-8558 (JP); FUKAZAWA, Takuya, Kita-ku Okayama-shi Okayama 700-8558 (JP)
(74) Representative: Zwicker, Jörk
(86) International application number: PCT/JP2009/053256
(87) International publication number: WO 2009/125626

(57) **Abstract**

Provided is a promoter showing transcriptional activity in a mesothelioma-specific manner and showing low transcriptional activity in other kinds of cancer cells and normal cells including mesothelium. Also provided are applications of the promoter, and more specifically, a gene therapy vector and a therapeutic agent for mesothelioma each including the promoter. The promoter includes a CRI1 gene-derived promoter, which is one kind of mesothelioma markers. The use of a vector including a cell death-inducing gene or a cell lysis-inducing gene as a transgene and carrying the CRI1 gene-derived promoter upstream of the transgene can induce a cell death or cell lysis action in a mesothelioma-specific manner. That is, the gene therapy vector and the therapeutic agent for mesothelioma each include a virus vector carrying the CRI1 gene-derived promoter.

SEQUENCE LISTING

<110>  National University Corporation Okayama University

<120>  Mesothelioma specific transferred Promoter and Use thereof

<130>  535-12

<150>  09 731 254.0
<151>  2009-02-24

<150>  JP 2008-104070
<151>  2008-04-11

<160>  14

<170>  PatentIn version 3.1

<210>  1
<211>  2670
<212>  DNA
<213>  Homo sapiens

<400>  1
gctagcacca caactagatg aactgatgaa atatcagagg ataatgaggc acaattgtta      60

gaaaggaaca atgctttttt gtgtgtgata tattttgcag gaggatgtga atgaggatat     120

gctatatgca cttttgttgc caaccagcac cacagatgca gaactattca agtctttgaa     180

tgattacata tcaggaaagt ggaatcagtc attttgcatc ggtatatgca tgggtggagc     240

agctgccatg actggatggc attttggttt cactactcag gtcaaagagg tcgcttctga     300

atgtgagtct atgcaccatg tcatccatag agaaatgatg gctagtcaga aaatgtcacc     360

tgaacttaac aacattttgc agtatgtgat taaaattatc aaccatatta tacatgccct     420

taactcacat ctgtttgtgc agctctgtga ggagatggat gcagagcaca cacgtcttct     480

cttacacaca gaagtgagat ggctttctaa aggtagatca ctggccagag tttttgagtt     540

aagagagctg cttcagagat ttctttgtag aaaaacagtc accattggcg gcacatttca     600

gtgacacaga atgggtcaca aaaaaacttg cttacttgtg tgacatattc aacctgctca     660

gtgaactctg tcacttcagg gacgaacaac aactgtgttc aacttggcag ataaggtggc     720

tgcattcaaa gccaaactgg aatcatgcgg gtgacaaatg aacactggga tttctgacat     780

ttcaaacatt agcagagatt ttgaaagaga ctgagccagg gtcttctttc tcccagctgg     840

tgcatgatca cctatctcag ctttcaaaat attttgagca ttacttcctg tattagtcat     900

ggttctctag aagcacagaa ctaatggaat atatacatat atatatacac acacacacac     960

acgtatatac acatacacac atacatatac acatatatat gtatatatat gtaaagggga    1020

gttcactaag tattaactca catgatcaca aggtcctaca ataggctgtc tgcaggctaa    1080

ggagagagga gagccagtcc gagttctgaa actgaagaac ttgggaatcc aatgttcgag    1140

ggcaggaagc atccaacaca ggagaaagat ttaggctggg aggctaggcc agtctctctt    1200

ttcacatttt tctgcctgct tatattctag ccaagctggc agctgattag attgtgccca    1260

accagattaa gggtgggtct gcctttcgca gcccactgac tcaaatgtta atctcctttg    1320

gcaacaccct cacagacaca ccaatgatca atacttgtat ccttcaatcc aatcaagttg 1380

acactcagta ttaaccatca cacttctcat ccacaaaaga cggaatggat ctgtgaccca 1440

tttgtgaata agccaggtga atccactttg tccacgctag aagaggatca actgcttgat 1500

attgcaaatg acggtggcct taaaagtatg tttgagacaa cttcaaatct ccatacgttc 1560

tcgattaaag tcaaggcgga acatcctgag attgccacaa aagtactgaa aagcctcctt 1620

ccattttcaa catcctatct ttgtgaggca ggattttcca cagtaacagc aaccaaaatg 1680

agattacaga atagaccgga cataaggaac acacctcggg tgtcactgtg tctcatcacc 1740

cccagatggg accatctagt tgcaggaaaa caagctcagg gctcccattg attctacatt 1800

atggtgagtt gtataattct acataactat aatgtaataa taatagaaat aaagtgcaga 1860

ataaatgaaa tgtgcttgaa tcctgtaagt atacacagtc atataattca tataagtata 1920

tacaacttaa aacctgaggc ttttaagttg aaggtaaaaa caggtaaaac atgtttttaa 1980

aaaacacaca aaatagtatt ttgctgtcaa tacataaaga tacatgtaaa tgcaaataaa 2040

aagaattcaa agaatacaca ccgcattgct aatagtggaa agatacctcc caggcgaaaa 2100

gaaatgggac caagtatttc ttcactataa ctaaaaaaaa aaaaagtaca atatatctgt 2160

tgtttgtgaa tttaactttt taaatgaaaa cactaaaagg acaaaatgga ttagttctta 2220

atatgctgat ctgtggttat ggatgttgtc tggtttggag acgtggattt acaagtcatc 2280

agtacatcgg taaattgccc aggggaagag gaaggggggg aaggagcatt aaatgttacg 2340

aaggccatgt aaggttattt tccggtttgc agcattacaa gcagcagtag ggttaagtaa 2400

cacagaaact gttacgctct tgcatgcagg gtccctagat acgaagttcg aatcgatgga 2460

aacgtagctc aaaaggcgac gccaacaccc cggagaaaac actgagctac tccaaccaca 2520

gtggcgcgcc aagtaggagg cggtacctga ggaccacgcc tgcgcgcggg gttacgcaag 2580

cgcgcagcct ttgcgcacgc gcacgaacgc acggccgcgc agcatctgtc ttgctggaag 2640

ctttttccta gaggttgagc ggtttgcaca 2670

<210> 2
<211> 1933
<212> DNA
<213> Homo sapiens

<400> 2
tggaatcatg cgggtgacaa atgaacactg ggatttctga catttcaaac attagcagag 60

attttgaaag agactgagcc agggtcttct ttctcccagc tggtgcatga tcacctatct 120

cagctttcaa aatattttga gcattacttc ctgtattagt catggttctc tagaagcaca 180

gaactaatgg aatatataca tatatatata cacacacaca cacacgtata tacacataca 240

cacatacata tacacatata tatgtatata tatgtaaagg ggagttcact aagtattaac 300

tcacatgatc acaaggtcct acaataggct gtctgcaggc taaggagaga ggagagccag 360

tccgagttct gaaactgaag aacttgggaa tccaatgttc gagggcagga agcatccaac 420

acaggagaaa gatttaggct gggaggctag gccagtctct cttttcacat ttttctgcct 480

gcttatattc tagccaagct ggcagctgat tagattgtgc ccaaccagat taagggtggg 540
tctgcctttc gcagcccact gactcaaatg ttaatctcct ttggcaacac cctcacagac 600
acaccaatga tcaatacttg tatccttcaa tccaatcaag ttgacactca gtattaacca 660
tcacacttct catccacaaa agacggaatg gatctgtgac ccatttgtga ataagccagg 720
tgaatccact ttgtccacgc tagaagagga tcaactgctt gatattgcaa atgacggtgg 780
ccttaaaagt atgtttgaga caacttcaaa tctccatacg ttctcgatta aagtcaaggc 840
ggaacatcct gagattgcca caaaagtact gaaaagcctc cttccatttt caacatccta 900
tctttgtgag gcaggatttt ccacagtaac agcaaccaaa atgagattac agaatagacc 960
ggacataagg aacacacctc gggtgtcact gtgtctcatc acccccagat gggaccatct 1020
agttgcagga aaacaagctc agggctccca ttgattctac attatggtga gttgtataat 1080
tctacataac tataatgtaa taataataga aataaagtgc agaataaatg aaatgtgctt 1140
gaatcctgta agtatacaca gtcatataat tcatataagt atatacaact taaaacctga 1200
ggcttttaag ttgaaggtaa aaacaggtaa aacatgtttt taaaaaacac acaaaatagt 1260
attttgctgt caatacataa agatacatgt aaatgcaaat aaaaagaatt caaagaatac 1320
acaccgcatt gctaatagtg gaaagatacc tcccaggcga aaagaaatgg gaccaagtat 1380
ttcttcacta taactaaaaa aaaaaaaagt acaatatatc tgttgtttgt gaatttaact 1440
ttttaaatga aaacactaaa aggacaaaat ggattagttc ttaatatgct gatctgtggt 1500
tatggatgtt gtctggtttg gagacgtgga tttacaagtc atcagtacat cggtaaattg 1560
cccaggggaa gaggaagggg gggaaggagc attaaatgtt acgaaggcca tgtaaggtta 1620
ttttccggtt tgcagcatta caagcagcag tagggttaag taacacagaa actgttacgc 1680
tcttgcatgc agggtcccta gatacgaagt tcgaatcgat ggaaacgtag ctcaaaaggc 1740
gacgccaaca ccccggagaa aacactgagc tactccaacc acagtggcgc gccaagtagg 1800
aggcggtacc tgaggaccac gcctgcgcgc ggggttacgc aagcgcgcag cctttgcgca 1860
cgcgcacgaa cgcacggccg cgcagcatct gtcttgctgg aagctttttc ctagaggttg 1920
agcggtttgc aca 1933

<210> 3
<211> 1768
<212> DNA
<213> Homo sapiens

<400> 3
ttctctagaa gcacagaact aatggaatat atacatatat atatacacac acacacacac 60
gtatatacac atacacacat acatatacac atatatatgt atatatatgt aaaggggagt 120
tcactaagta ttaactcaca tgatcacaag gtcctacaat aggctgtctg caggctaagg 180
agagaggaga gccagtccga gttctgaaac tgaagaactt gggaatccaa tgttcgaggg 240
caggaagcat ccaacacagg agaaagattt aggctgggag gctaggccag tctctctttt 300

cacatttttc tgcctgctta tattctagcc aagctggcag ctgattagat tgtgcccaac 360
cagattaagg gtgggtctgc ctttcgcagc ccactgactc aaatgttaat ctcctttggc 420
aacaccctca cagacacacc aatgatcaat acttgtatcc ttcaatccaa tcaagttgac 480
actcagtatt aaccatcaca cttctcatcc acaaaagacg gaatggatct gtgacccatt 540
tgtgaataag ccaggtgaat ccactttgtc cacgctagaa gaggatcaac tgcttgatat 600
tgcaaatgac ggtggcctta aaagtatgtt tgagacaact tcaaatctcc atacgttctc 660
gattaaagtc aaggcggaac atcctgagat tgccacaaaa gtactgaaaa gcctccttcc 720
attttcaaca tcctatcttt gtgaggcagg attttccaca gtaacagcaa ccaaaatgag 780
attacagaat agaccggaca taaggaacac acctcgggtg tcactgtgtc tcatcacccc 840
cagatgggac catctagttg caggaaaaca agctcagggc tcccattgat tctacattat 900
ggtgagttgt ataattctac ataactataa tgtaataata atagaaataa agtgcagaat 960
aaatgaaatg tgcttgaatc ctgtaagtat acacagtcat ataattcata taagtatata 1020
caacttaaaa cctgaggctt ttaagttgaa ggtaaaaaca ggtaaaacat gtttttaaaa 1080
aacacacaaa atagtatttt gctgtcaata cataaagata catgtaaatg caaataaaaa 1140
gaattcaaag aatacacacc gcattgctaa tagtggaaag atacctccca ggcgaaaaga 1200
aatgggacca agtatttctt cactataact aaaaaaaaaa aaagtacaat atatctgttg 1260
tttgtgaatt taacttttta aatgaaaaca ctaaaaggac aaaatggatt agttcttaat 1320
atgctgatct gtggttatgg atgttgtctg gtttggagac gtggatttac aagtcatcag 1380
tacatcggta aattgcccag gggaagagga agggggggaa ggagcattaa atgttacgaa 1440
ggccatgtaa ggttattttc cggtttgcag cattacaagc agcagtaggg ttaagtaaca 1500
cagaaactgt tacgctcttg catgcagggt ccctagatac gaagttcgaa tcgatggaaa 1560
cgtagctcaa aaggcgacgc caacaccccg gagaaaacac tgagctactc caaccacagt 1620
ggcgcgccaa gtaggaggcg gtacctgagg accacgcctg cgcgcggggt tacgcaagcg 1680
cgcagccttt gcgcacgcgc acgaacgcac ggccgcgcag catctgtctt gctggaagct 1740
ttttcctaga ggttgagcgg tttgcaca 1768

<210> 4
<211> 1671
<212> DNA
<213> Homo sapiens

<400> 4
tgtatatata tgtaaagggg agttcactaa gtattaactc acatgatcac aaggtcctac 60
aataggctgt ctgcaggcta aggagagagg agagccagtc cgagttctga aactgaagaa 120
cttgggaatc caatgttcga gggcaggaag catccaacac aggagaaaga tttaggctgg 180
gaggctaggc cagtctctct tttcacattt ttctgcctgc ttatattcta gccaagctgg 240
cagctgatta gattgtgccc aaccagatta agggtgggtc tgcctttcgc agcccactga 300
ctcaaatgtt aatctccttt ggcaacaccc tcacagacac accaatgatc aatacttgta 360

tccttcaatc caatcaagtt gacactcagt attaaccatc acacttctca tccacaaaag 420

acggaatgga tctgtgaccc atttgtgaat aagccaggtg aatccacttt gtccacgcta 480

gaagaggatc aactgcttga tattgcaaat gacggtggcc ttaaaagtat gtttgagaca 540

acttcaaatc tccatacgtt ctcgattaaa gtcaaggcgg aacatcctga gattgccaca 600

aaagtactga aaagcctcct tccattttca acatcctatc tttgtgaggc aggattttcc 660

acagtaacag caaccaaaat gagattacag aatagaccgg acataaggaa cacacctcgg 720

gtgtcactgt gtctcatcac ccccagatgg gaccatctag ttgcaggaaa acaagctcag 780

ggctcccatt gattctacat tatggtgagt tgtataattc tacataacta taatgtaata 840

ataatagaaa taaagtgcag aataaatgaa atgtgcttga atcctgtaag tatacacagt 900

catataattc atataagtat atacaactta aaacctgagg cttttaagtt gaaggtaaaa 960

acaggtaaaa catgttttta aaaaacacac aaaatagtat tttgctgtca atacataaag 1020

atacatgtaa atgcaaataa aaagaattca aagaatacac accgcattgc taatagtgga 1080

aagatacctc ccaggcgaaa agaaatggga ccaagtattt cttcactata actaaaaaaa 1140

aaaaaagtac aatatatctg ttgtttgtga atttaacttt ttaaatgaaa acactaaaag 1200

gacaaaatgg attagttctt aatatgctga tctgtggtta tggatgttgt ctggtttgga 1260

gacgtggatt tacaagtcat cagtacatcg gtaaattgcc caggggaaga ggaagggggg 1320

gaaggagcat taaatgttac gaaggccatg taaggttatt ttccggtttg cagcattaca 1380

agcagcagta gggttaagta acacagaaac tgttacgctc ttgcatgcag ggtccctaga 1440

tacgaagttc gaatcgatgg aaacgtagct caaaaggcga cgccaacacc ccggagaaaa 1500

cactgagcta ctccaaccac agtggcgcgc caagtaggag gcggtacctg aggaccacgc 1560

ctgcgcgcgg ggttacgcaa gcgcgcagcc tttgcgcacg cgcacgaacg cacggccgcg 1620

cagcatctgt cttgctggaa gctttttcct agaggttgag cggtttgcac a 1671

<210> 5
<211> 1167
<212> DNA
<213> Homo sapiens

<400> 5
gcaaatgacg gtggccttaa aagtatgttt gagacaactt caaatctcca tacgttctcg 60

attaaagtca aggcggaaca tcctgagatt gccacaaaag tactgaaaag cctccttcca 120

ttttcaacat cctatctttg tgaggcagga ttttccacag taacagcaac caaaatgaga 180

ttacagaata gaccggacat aaggaacaca cctcgggtgt cactgtgtct catcaccccc 240

agatgggacc atctagttgc aggaaaacaa gctcagggct cccattgatt ctacattatg 300

gtgagttgta taattctaca taactataat gtaataataa tagaaataaa gtgcagaata 360

aatgaaatgt gcttgaatcc tgtaagtata cacagtcata taattcatat aagtatatac 420

aacttaaaac ctgaggcttt taagttgaag gtaaaaacag gtaaaacatg tttttaaaaa 480

acacacaaaa tagtattttg ctgtcaatac ataaagatac atgtaaatgc aaataaaaag 540

aattcaaaga atacacaccg cattgctaat agtggaaaga tacctcccag gcgaaaagaa 600

atgggaccaa gtatttcttc actataacta aaaaaaaaaa aagtacaata tatctgttgt 660

ttgtgaattt aactttttaa atgaaaacac taaaaggaca aaatggatta gttcttaata 720

tgctgatctg tggttatgga tgttgtctgg tttggagacg tggatttaca agtcatcagt 780

acatcggtaa attgcccagg ggaagaggaa gggggggaag gagcattaaa tgttacgaag 840

gccatgtaag gttattttcc ggtttgcagc attacaagca gcagtagggt taagtaacac 900

agaaactgtt acgctcttgc atgcagggtc cctagatacg aagttcgaat cgatggaaac 960

gtagctcaaa aggcgacgcc aacaccccgg agaaaacact gagctactcc aaccacagtg 1020

gcgcgccaag taggaggcgg tacctgagga ccacgcctgc gcgcggggtt acgcaagcgc 1080

gcagcctttg cgcacgcgca cgaacgcacg gccgcgcagc atctgtcttg ctggaagctt 1140

tttcctagag gttgagcggt ttgcaca 1167

<210> 6
<211> 850
<212> DNA
<213> Homo sapiens

<400> 6
acataactat aatgtaataa taatagaaat aaagtgcaga ataaatgaaa tgtgcttgaa 60

tcctgtaagt atacacagtc atataattca tataagtata tacaacttaa aacctgaggc 120

ttttaagttg aaggtaaaaa caggtaaaac atgtttttaa aaaacacaca aaatagtatt 180

ttgctgtcaa tacataaaga tacatgtaaa tgcaaataaa aagaattcaa agaatacaca 240

ccgcattgct aatagtggaa agatacctcc caggcgaaaa gaaatgggac caagtatttc 300

ttcactataa ctaaaaaaaa aaaaagtaca atatatctgt tgtttgtgaa tttaactttt 360

taaatgaaaa cactaaaagg acaaaatgga ttagttctta atatgctgat ctgtggttat 420

ggatgttgtc tggtttggag acgtggattt acaagtcatc agtacatcgg taaattgccc 480

aggggaagag gaaggggggg aaggagcatt aaatgttacg aaggccatgt aaggttattt 540

tccggtttgc agcattacaa gcagcagtag ggttaagtaa cacagaaact gttacgctct 600

tgcatgcagg gtccctagat acgaagttcg aatcgatgga aacgtagctc aaaaggcgac 660

gccaacaccc cggagaaaac actgagctac tccaaccaca gtggcgcgcc aagtaggagg 720

cggtacctga ggaccacgcc tgcgcgcggg gttacgcaag cgcgcagcct ttgcgcacgc 780

gcacgaacgc acggccgcgc agcatctgtc ttgctggaag ctttttccta gaggttgagc 840

ggtttgcaca 850

<210> 7
<211> 651
<212> DNA
<213> Homo sapiens

<400> 7

atacatgtaa atgcaaataa aaagaattca aagaatacac accgcattgc taatagtgga 60

aagatacctc ccaggcgaaa agaaatggga ccaagtattt cttcactata actaaaaaaa 120

aaaaaagtac aatatatctg ttgtttgtga atttaacttt ttaaatgaaa acactaaaag 180

gacaaaatgg attagttctt aatatgctga tctgtggtta tggatgttgt ctggtttgga 240

gacgtggatt tacaagtcat cagtacatcg gtaaattgcc caggggaaga ggaagggggg 300

gaaggagcat taaatgttac gaaggccatg taaggttatt ttccggtttg cagcattaca 360

agcagcagta gggttaagta acacagaaac tgttacgctc ttgcatgcag ggtccctaga 420

tacgaagttc gaatcgatgg aaacgtagct caaaaggcga cgccaacacc ccggagaaaa 480

cactgagcta ctccaaccac agtggcgcgc caagtaggag gcggtacctg aggaccacgc 540

ctgcgcgcgg ggttacgcaa gcgcgcagcc tttgcgcacg cgcacgaacg cacggccgcg 600

cagcatctgt cttgctggaa gctttttcct agaggttgag cggtttgcac a 651

<210> 8
<211> 1013
<212> DNA
<213> Homo sapiens

<400> 8
atacatgtaa atgcaaataa aaagaattca aagaatacac accgcattgc taatagtgga 60

aagatacctc ccaggcgaaa agaaatggga ccaagtattt cttcactata actaaaaaaa 120

aaaaaagtac aatatatctg ttgtttgtga atttaacttt ttaaatgaaa acactaaaag 180

gacaaaatgg attagttctt aatatgctga tctgtggtta tggatgttgt ctggtttgga 240

gacgtggatt tacaagtcat cagtacatcg gtaaattgcc caggggaaga ggaagggggg 300

gaaggagcat taaatgttac gaaggccatg taaggttatt ttccggtttg cagcattaca 360

agcagcagta gggttaagta acacagaaac tgttacgctc ttgcatgcag ggtccctaga 420

tacgaagttc gaatcgatgg aaacgtagct caaaaggcga cgccaacacc ccggagaaaa 480

cactgagcta ctccaaccac agtggcgcgc caagtaggag gcggtacctg aggaccacgc 540

ctgcgcgcgg ggttacgcaa gcgatatgct gatctgtggt tatggatgtt gtctggtttg 600

gagacgtgga tttacaagtc atcagtacat cggtaaattg cccaggggaa gaggaagggg 660

gggaaggagc attaaatgtt acgaaggcca tgtaaggtta ttttccggtt tgcagcatta 720

caagcagcag tagggttaag taacacagaa actgttacgc tcttgcatgc agggtcccta 780

gatacgaagt tcgaatcgat ggaaacgtag ctcaaaaggc gacgccaaca ccccggagaa 840

aacactgagc tactccaacc acagtggcgc gccaagtagg aggcggtacc tgaggaccac 900

gcctgcgcgc ggggttacgc aagcgcgcag cctttgcgca cgcgcacgaa cgcacggccg 960

cgcagcatct gtcttgctgg aagctttttc ctagaggttg agcggtttgc aca 1013

<210> 9
<211> 380
<212> DNA
<213> Homo sapiens

<400>  9
taaattgccc aggggaagag gaaggggggg aaggagcatt aaatgttacg aaggccatgt      60

aaggttattt tccggtttgc agcattacaa gcagcagtag ggttaagtaa cacagaaact     120

gttacgctct tgcatgcagg gtccctagat acgaagttcg aatcgatgga aacgtagctc     180

aaaaggcgac gccaacaccc cggagaaaac actgagctac tccaaccaca gtggcgcgcc     240

aagtaggagg cggtacctga ggaccacgcc tgcgcgcggg gttacgcaag cgcgcagcct     300

ttgcgcacgc gcacgaacgc acggccgcgc agcatctgtc ttgctggaag ctttttccta     360

gaggttgagc ggtttgcaca                                                 380

<210>  10
<211>  222
<212>  DNA
<213>  Homo sapiens

<400>  10
cgaatcgatg gaaacgtagc tcaaaaggcg acgccaacac cccggagaaa acactgagct      60

actccaacca cagtggcgcg ccaagtagga ggcggtacct gaggaccacg cctgcgcgcg     120

gggttacgca agcgcgcagc ctttgcgcac gcgcacgaac gcacggccgc gcagcatctg     180

tcttgctgga agctttttcc tagaggttga gcggtttgca ca                        222

<210>  11
<211>  292
<212>  DNA
<213>  Homo sapiens

<400>  11
cgaatcgatg gaaacgtagc tcaaaaggcg acgccaacac cccggagaaa acactgagct      60

actccaacca cagtggcgcg ccaagtagga ggcggtacct gaggaccacg cctgcgcgcg     120

gggttacgca agcgcaacca cagtggcgcg ccaagtagga ggcggtacct gaggaccacg     180

cctgcgcgcg gggttacgca agcgcgcagc ctttgcgcac gcgcacgaac gcacggccgc     240

gcagcatctg tcttgctgga agctttttcc tagaggttga gcggtttgca ca             292

<210>  12
<211>  2248
<212>  DNA
<213>  Homo sapiens

<400>  12
ggggctgtgc agcgcagtgg ttaagaactt gtgttctgga gacagtatct gtcttagcct      60

ttcccgttgg cctcccgttg gggtcagccc ctccaggatc cattcattag ctaccccagg     120

ggaggtgatg ctgactgaga aatttcttgc caagccaacc actgatcact ggctccatga     180

agggcggcag gagccctaac tcatttaatc ctcagaacaa ttatattatt attattatta     240

ctattattag agacagagtc tcgctctgtc gcccaggctg ggtgcagtgg gtgcgatctt     300

ggctcactgc aacctttccg cctcccgggt tcaagcgatt ctcctgcctc agcctcccaa     360

gtagctggga ttacaagcat gcaccgccac gcctggctaa tttttttttt tttttttttt     420

tttttttttt tagtagagac tgggtttcac catgttggcc aggatgatct caatctctta 480
acctcgtgat ctgcccacct cggcctccca aagtgctagg attacaggca tgagccacgg 540
cacccagcca acaattctaa aaggcgtatg cccacatgcc cattttgcag aagagaaaaa 600
ctgaggcaga gacaggttat ataatttgct caaggtcaca cagcttattc tgggattcac 660
acccaagcag cctgattctt gagtgcctac tcttaaccac tgagtcatcc tgcctcccta 720
gggactttgg tgaatctctc agcaaggtgg atgggtacct ctaaagctag cttttgatga 780
cttctccccc cagtctgttc ctgcaagtaa ctctaaacac attcacccta ggcaaagaac 840
aagtggtttt ggaacctgca ttctctattg cccctttgag actcgctctg ggcttggccc 900
tctctccaag ggacaaccct ctctctgtct cctcctgggt tcccctctcc cccagcgacc 960
tccttttcat tctcacttac ctccttgcct ttcactctcc tcttggaagg tggttcagct 1020
aacactcatt agcacatgtt aaagatcgcc aattcatgtc tcagtcactc cgtagcagga 1080
gaggggagaa aggagggatg atgctgtcac tctgtgggta atgtgtctta ccttaccaca 1140
ccacaaggcc aggacagacc cctaagggct cagaccgcag gagagaatgg ggagagggcc 1200
cagctccctg ctggggagtc ctgtctgctg ccctcaggat gtgcgctcag tagctgcgtc 1260
tattttctct gagaccagct cagaacatcc ccaagacaga gttggacgtt gttctctgtc 1320
cactggagca ggcacattcc cacgatgtcc ctaggtgggc gtggttaaga cctggtacgg 1380
gttgatctag ttctgccacc ccctggccaa atgtctaaaa gccccccaga gcatggccag 1440
cgtgaggcag gtaccagggg tggagggagg ctccgagggg acggatatac gaagacccaa 1500
acagacagtg gaagcccccc acccccaccc cacaccactt ccatcggaat cctcccgggg 1560
cactgctgat tccagctgct ccccactaaa gccttgagaa ctcttggctg ctctgcaaga 1620
ctgagcccca tgaaggagcc acgtgcggcg tggaaagagt gctgagttca aattgtagcc 1680
ctgccactaa tttgctgggc cagtcactta atcatctgaa gtcacaagta cctcatcaga 1740
aaagtggtcc cagctcttcc tgctgtggaa ggatcagaag agaggaggca cgacagagac 1800
ctagtgaact ccgaagcccg agtgctaaat atttgtcaag tttgtgttag tattactatt 1860
agtgttgtta ctgctgttat tattattgct actgccagca ataataagtg gtggatgtac 1920
tcaagacggt cgggagggaa ggcaagggca gcctctccct cattttcacc gaaaatcctc 1980
cgggtgtccc tggccccgcg ccgaggggtc tcagcgcaga ggtaagggcc ctctaggagt 2040
ccgggccgag cctctcgcgc cgccgccccc gccgcgccgc gccccggtcg gattccctga 2100
gcgcgcgcgc ccccttctgg cggccgggcg caggcgcagg ctccagagcg tatataaggg 2160
cagcgtggcg cacaacccag cgcgagtgcc agagcccagc cggcgcggag cgggagcggt 2220
gcaggctgag gtctccgagc ggctcgcc 2248

<210> 13
<211> 1926
<212> DNA
<213> Homo sapiens

<400> 13
tttgtctcga gagtcctttc tccactcaaa aaaccaaacg cgcgagcccc gcgaaaggtt 60
tagggataga tcgtgtggga gaggactgag cagagagcgt gggggcagtg tcttgtagaa 120
tctttctttt cttaataata attttaaaag cttctgagtg gagacgacgc aaagtcaagc 180
agcaaaggtg gcctgggagg caagcggagg gctcaagtgc cgcatcttta ccctcagggt 240
ctcctgcgcc tacgggatgc gcattcccaa gaagtgcgcc cttcgagtaa gtcctgggcc 300
cgcacacact tcgggtccgc agccagaatt taatggcgac aacgtttatg caatgcaagc 360
taaaaaccaa agcgtaaaaa attactatgt catttattga aacgccattc tttgtcaaac 420
tgcaactact ttgcttcaca taagtttggc tggaaagctt gcagccccag cccgggccag 480
ccaggtacag gaggccggac tgcaaccggt tgcttccctc ccgtcgcgcc tggccgtccc 540
acgctgcgcc gtcgctgctg cctcctggcg cccctgggat tttatacgca cctctgaaac 600
acgctccgct ccggcccccg gttcttctcc ttgcctaggg gttgtttccc aatagatact 660
gactccttta gaagatccaa aaaccaaacc aaaacacccc ctacccgccc caaacacctg 720
ctctggggcg cgggggctgc caaacagaga ctagacgaag ggagtcagat ttagcgaagc 780
tcttcgagct cccaaagatt cgaacactaa ctcgcgcccg tgggccgatg gaggttctcc 840
ctactccact ccttggtccc cttaactggc ttccgcctcc tggtcaatca ctgagcaacc 900
agaatggtat cctcgaccag ggccacaggc agtgctcggc ggagtggctc caggagttac 960
ccgctccctg ccgggcttcg tatccaaacc ctccccttca cccctcctcc ccaaactggg 1020
cgccaggatg ctccggccgg aatatacgca ggctttgggc gtttgcccaa gggttttctt 1080
ccctcctaaa ctagccgctg ttttcccggc ttaaccgtag aagaattaga tattcctcac 1140
tggaaaggga aactaagtgc tgctgactcc aattttaggt aggcggcaac cgccttccgc 1200
ctggcgcaaa cctcaccaag taaacaacta ctagccgatc gaaatacgcc cggcttataa 1260
ctggtgcaac tcccggccac ccaactgagg gacgttcgct ttcagtcccg acctctggaa 1320
cccacaaagg gccacctctt tccccagtga ccccaagatc atggccactc ccctacccga 1380
cagttctaga agcaagagcc agactcaagg gtgcaaagca agggtatacg cttctttgaa 1440
gcttgactga gttctttctg cgctttcctg aagttcccgc cctcttggag cctacctgcc 1500
cctccctcca aaccactctt ttagattaac aaccccatct ctactcccac cgcattcgac 1560
cctgcccgga ctcactgctt acctgaacgg actctccagt gagacgaggc tcccacactg 1620
gcgaaggcca agaaggggag gtggggggag ggttgtgcca caccggccag ctgagagcgc 1680
gtgttgggtt gaagaggagg gtgtctccga gagggacgct ccctcggacc cgccctcacc 1740
ccagctgcga gggcgccccc aaggagcagc gcgcgctgcc tggccgggct tgggctgctg 1800
agtgaatgga gcggccgagc ctcctggctc ctcctcttcc ccgcgccgcc ggcccctctt 1860
atttgagctt tgggaagctg agggcagcca ggcagctggg gtaaggagtt caaggcagcg 1920
cccaca 1926

<210> 14
<211> 2354
<212> DNA
<213> Homo sapiens

<400> 14
ggtcaggctt gtgctcccgg gagtcctgtc tgggctgcgt ggccaccatc cagagcctgc 60

tgacctgcga ctgggggggc cagtgctccc tgggtttcag cacctgagaa tcagagtggg 120

atcccgtgaa acctgggccc aggctcccac ccacgcccca cacccaccca gggaagccat 180

gaaacctggg cccgggctcc tacacatgcc ccacacccac ccagggcagc cgtgaaacct 240

gggcccgggc tcccaccctc gcccaccgag ggcagctttg ccttcctggg catccctcct 300

cccccaggcc tggcccgctg cctgtccaag gctcctgtgc ggggtctcca cccacacatt 360

cctggggcgt gaggcgccac cactccctgc tgccccgggc aaagccgtca tttgttccct 420

ttgacggccc gggaggctgc caggctctcc acccccactt cccaattgag gaaaccgagg 480

cagaggaggc tcaggtgtgg ccaatcaccc tgcacatcag agttaccctg ggcagggccc 540

actgagacct gggaggggcc actcgggacc tggagggctg ggggctgccc gggcgttagg 600

ggtaaagctc cctacccaac tgcgcagaag gcctcagagg cctgggggct gggcttcccc 660

tttcacatcg ccctttagag gcccacgtgt gggcattggc ccgcgatctg aaaggggctg 720

tcctgttcct catgggcgct gccagcgcca cgcactcctc tttctgcctg gccggccact 780

cccgtctgct gtgacgcgcg gacagagagc taccggtgga cccacggtgc ctccctccct 840

gggatctgta agtaacaacc tttgagctct tcctgttgtg gtgtggatgg aatctgcacc 900

ttccatctgg agaactgggg ccgccccagg ccgggcttcc agcaccagag cgatggtcag 960

gcttcagctg gacgcagatt tcaccccgca gggcacacgc agacccattt gtttggaagc 1020

ggcagtccta aggcagtgca ggtgcctcca cggccccagc ccagctctcc tgcaggtgta 1080

aaggatgcag attccagggc cccccatggc ctgcagaggc ccctcagagc aggaagggcc 1140

gggctctcgg ggagccccca tcccaggtgc tgaggtcctt gggtcacatg gagctggggt 1200

ggggagtgcc caggtcccct gtgatccgat ggccacgttt ccctcctggc ttcacgcacc 1260

ccaagggctg ggggtcccac tcactgccca ttttctggct gtgggggggg tctgttttct 1320

tccaccttct caaggtgttc agagcctcag gggcctcccc acttcctctg gggcctgggg 1380

gagtagagtc tgtcacccgg aggctgtgtg cctccccgcg acattcccca gtggccgggc 1440

cgaccaggca ggctctgcct gtggatgggg ctgctgagca tgagcgtgtg acccccattt 1500

ctgagctgtg ggtggaccca ccgggcctgg gccaaggttt tcaggaggca gctctattct 1560

actccgccat ggtggcctcc ctgaaagggg tgagtgtaga agccagttcc ctcttctgcg 1620

agccccaccc ctaccccagg aggacaattc ttgttcaggg agggtctccc cacccacttc 1680

cccaccccac agtggcccac aggccccacc ctagagagta caaggggctc cccaggctgc 1740

tcactggccc agccccgccc cacctggact gcacctgaaa atgggctcag agaggccaag 1800

tggcccaaaa agacgctgct gggtgggaag gggcggtggc ctctgtgccc gcagtgcccc 1860

tcctgcctca agggtgttgt ctgcctggca gagcctgggg tgttcacaaa gcccaggcac 1920
ctgcagctga gggcagggga gagggaaggg agccacatcc aggcgacggg gctgctcgtc 1980
ctcctgtgcg agagtgggga gactcaggcc agcccaagtg ggcggcggcc ccggttgctt 2040
gatctaagct ctgctcacac tgccctgccc ttctgggaga ggggtgggcg ccaactgact 2100
cctgggctgt ctgggctggg gaccgggatc tcagacccag cccctcccct ggacaggagg 2160
agccagtcca ggggacagag ggctcagtgg ctggagggca gggccagggt gcggacacaa 2220
gctgcaggta ccacagaagt ttgctctggg agcccctcct gggcccatgt ggccccaggc 2280
tggcccagga cagaggcgtg gggtgggagc caggggtccc catcctgagt cactgccctc 2340
cacagacaca gacc 2354

## Description

### Technical Field

The present invention relates to a promoter showing transcriptional activity in a mesothelioma-specific manner and showing low transcriptional activity in other kinds of cancer cells and normal cells including mesothelium. The present invention also relates to applications of the promoter, and more specifically, to a gene therapy vector and a therapeutic agent for mesothelioma each including the promoter.

The present application claims the priority of Japanese Patent Application No. 2008-104070, the disclosure of which is incorporated herein by reference.

### Background Art

Thoracic organs such as lungs or heart and abdominal organs such as stomach, intestines, or liver are each surrounded by a membrane called pleura, peritoneum, pericardium, or the like. It is "mesothelium" that covers the surface of such membrane. Mesothelioma is a general term for mesothelial cell-derived tumors, and may be malignant or benign. Mesothelioma often develops in the pleura and also develops in the peritoneum, pericardium, and the like. Mesotheliomas that develop in the pleura, peritoneum, and pericardium are referred to as pleural mesothelioma, peritoneal mesothelioma, and pericardial mesothelioma, respectively. Mesothelioma is often discussed in relation to asbestos. In this case, the mesothelioma mainly refers to malignant pleural mesothelioma.

The risk of mesothelioma is increased by a higher level of asbestos exposure and a longer history of the exposure, and there is a long latency period between the asbestos exposure and the development of mesothelioma. It is said that mesothelioma has a latency period of around 20 years at least and about 40 years on average before the development. There is an indication that the incidence of lung cancer is increased several-fold to 50-fold in people with both risks of asbestos exposure and smoking. However, it is believed that mesothelioma has little association with smoking.

As methods for diagnosis of mesothelioma, there are exemplified image findings, cytodiagnosis of pleural fluid, tissue biopsies, and detection of tumor markers. In the image findings, extrapleural sign and pleural effusion are observed with X-rays in many cases, which are generally unilateral. Similar findings can also be obtained by thoracic CT. Further, an image showing the accumulation of FDG is obtained in FDG-PET. In the cytodiagnosis of pleural fluid, tumor cells are observed in some cases. The tissue biopsies are extremely important and provide a primary basis for definitive diagnosis. It has been reported that the expression of Wilms' tumor susceptibility gene 1 (WT1) (Non-patent Documents 1 to 3), calretinin (Non-patent Documents 4 and 5), mesothelin (Non-patent Document 5), or CREBBP/EP300 inhibitory protein 1 (CRI1) (Non-patent Document 6) as a mesothelioma marker is observed. It should be noted that Carim et al. reported that CRI1 was identified as C150RF13 by EST cluster analysis (Non-patent Document 7) and Gordon et al. reported that CRI1 was analyzed and expressed significantly in mesothelioma (Non-patent Document 6). However, there is no report on the pathogenicty of CRI1. The sequence of a gene encoding CRI1 has been registered with GenBank Accession No. NM_014335 and is also referred to as EP300 interacting inhibitor of differentiation 1 (EID1).

A method for treatment of mesothelioma also varies depending on the stage such as limited pleural mesothelioma (Stage I) or advanced pleural mesothelioma (Stage II, III, or IV). For example, limited pleural mesothelioma (Stage I) is treated by surgical therapy involving removing part of the pleura and its surrounding tissues. When a tumor is present in a wider range of the pleura, the tumor is treated by surgical therapy involving removing the pleura and its adjacent tissues in order to reduce the symptoms, and is further treated by radiotherapy and chemotherapy as the case may be. A method for treatment of advanced pleural mesothelioma (Stage II, III, or IV) varies depending on the stage, and for example, thoracentesis for removing fluid from the pleural cavity, and surgical therapy, radiotherapy, and chemotherapy are performed. Mesothelioma rarely metastasizes to other organs. However, mesothelioma has already progressed extensively at the time of diagnosis, and hence cannot be treated by a radical operation in many cases. Mesothelioma is said to show extremely poor prognosis and have a one-year survival rate of 50% and a two-year survival rate of 20%.

In recent years, many attempts have been made on gene therapy as one of methods for treatment of diseases. Further, there are various reports on vectors which may be used for such gene therapy, and the vectors are expected to be applied to anti-tumor agents (Patent Documents 1 and 2). Adenovirus vectors (also referred to as "Ad vectors") are exemplified as one kind of vectors used for gene therapy. At present, the Ad vectors used for the vectors for gene therapy are based on human Ad type 5 (or type 2) belonging to the sub-group C.

The Ad vectors are expected to be applied to various diseases as the vectors for gene therapy because of their excellent transgenic property. However, when the Ad vectors are locally administered to tumors, some of the Ad vectors may leak from the tumors into the general circulation. The expression of a gene in a site other than an affected site of interest may cause undesired adverse effects. For example, the use of a gene showing toxicity on cells expressing the gene may cause toxicity on not only tumors, i.e., mesothelioma but also tissues other than the tumors. It is conceivable that the expression of a desired gene in only cells or tissues of interest would lead to an effective gene therapy without any adverse effect.
Non-patent Document 1: Differentiation, 65: 89-96, 1999
Non-patent Document 2: Cancer Research, 61: 921-925, 2001
Non-patent Document 3: J. Pathol., 199: 479-487, 2003
Non-patent Document 4: Human Pathology, 34: 994-1000, 2003
Non-patent Document 5: Proc. Natl. Acad. Sci. USA, 93: 136-140, 1996
Non-patent Document 6: Am. J. Pathol., 166: 1827-1840, 2005
Non-patent Document 7: Cytogenet. Cell Genet., 88: 330-332, 2000
Patent Document 1: JP 2007-209328 A
Patent Document 2: JP 2007-190022 A

### Disclosure of the Invention

### Problems to be solved by the Invention

An object of the present invention is to provide a promoter showing transcriptional activity in a mesothelioma-specific manner and showing low transcriptional activity in other kinds of cancer cells and normal cells including mesothelium. Another object of the present invention is to provide applications of the promoter, and more specifically, to provide a gene therapy vector and a therapeutic agent for mesothelioma each including the promoter.

### Means for solving the Problems

The inventors of the present invention have intensively studied in order to solve the above-mentioned problems. As a result, the inventors have focused on a mesothelioma marker, and have succeeded in finding a mesothelioma marker-related promoter, which shows transcriptional activity in a mesothelioma-specific manner and shows no transcriptional activity in other kinds of cancer cells and normal cells including mesothelium. Thus, the present invention has been completed.

That is, the present invention includes the following:
1. a novel promoter, including a CREBBP/EP300 inhibitory protein 1 (CRI1) gene-derived promoter, in which the promoter shows transcriptional activity in a mesothelioma-specific manner;
2. a novel promoter according to the item 1, in which the CRI1 gene-derived promoter has a sequence selected from the region of -2586 to +84 in a CRI1 gene;
3. a novel promoter according to the item 1 or 2, in which the CRI1 gene-derived promoter has a sequence represented by any one of SEQ ID NOS: 1 to 11 in Sequence Listing;
4. a virus vector, including the novel promoter according to any one of the items 1 to 3;
5. a virus vector according to the item 4, in which the virus vector includes an adenovirus vector;
6. a virus vector according to the item 5, in which the adenovirus includes a conditionally replication-competent adenovirus;
7. a virus vector according to any one of the items 4 to 6, further including a cell death-inducing gene and/or a cell lysis-inducing gene downstream of the promoter;
8. a gene therapy vector for treatment of mesothelioma, including the virus vector according to any one of the items 4 to 7;
9. a therapeutic agent for mesothelioma, including the gene therapy vector for treatment of mesothelioma according to the item 8;
10. a virus vector according to any one of the items 4 to 6, further including a marker gene downstream of the promoter;
11. a virus vector according to the item 10, in which the marker gene includes a fluorescent protein-expressing gene;
12. a virus vector for inspection of mesothelioma, including the virus vector according to the item 10 or 11; and
13. a method for inspection of mesothelioma, including observing the presence or absence of the expression of a marker by using the virus vector for inspection of mesothelioma according to the item 12.

### Effects of the Invention

The novel promoter of the present invention showed significant transcriptional activity in mesothelioma and showed low transcriptional activity in other kinds of cancer cells and normal cells including mesothelium. Thus, the utilization of a cell death-inducing or cell lysis-inducing vector carrying the promoter can effectively induce a cell death or cell lysis action in a mesothelioma-specific manner. Further, an anti-tumor effect was confirmed in vivo as well. The results of in vitro and in vivo confirmation indicate that, when the vector is E1-deleted Ad, a gene encoding an E1 region is used as a transgene and incorporated into the vector together with the promoter of the present invention, which allows Ad to replicate in a mesothelioma-specific manner, leading to the disappearance of mesothelioma. In view of the foregoing, a therapeutic agent effective for mesothelioma can be provided.

### Brief Description of the Drawings

FIG. 1 is a diagram illustrating expression constructs produced by excising promoter regions from various mesothelioma marker genes, and allowing the regions to bind to firefly luciferase genes (Example 1).
FIG. 2 is a graph showing the transcriptional activity of various mesothelioma marker gene-derived promoters in mesothelioma or lung cancer cells (Example 1).
FIG. 3 is a graph showing the transcriptional activity of various mesothelioma marker gene-derived promoters in normal cells (Example 1).
FIG. 4 is a diagramillustrating expression constructs produced by excising promoter regions from a CRI1 gene, and allowing the regions to bind to firefly luciferase genes (Example 2).
FIG. 5 is a graph showing the transcriptional activity of the respective CRI1 gene-derived promoters in various cells (Example 2).

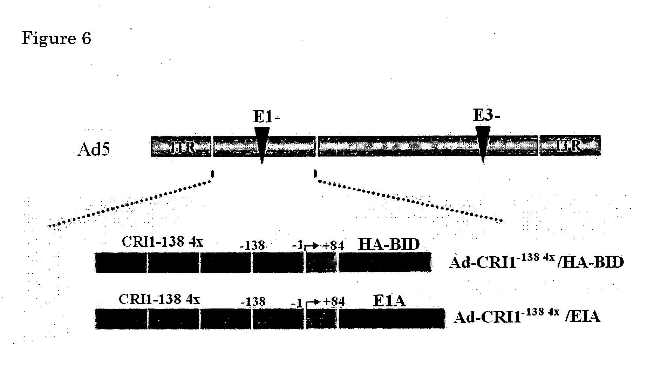

FIG. 6 is a schematic diagram illustrating Ad vectors carrying a promoter of the present invention and each transgene (Example 3).
FIGS. 7 are panels showing flow cytometric patterns in the case of infecting an Ad vector of the present invention to various cells (Experimental Example 1).
FIGS. 8 are graphs showing the measurement results of the number of viable cells in the case of infecting the Ad vector of the present invention to various cells (Experimental Example 2).
FIG. 9 is a graph showing the volume of tumor cells in the case of administering the Ad vector of the present invention to a mouse tumor model (Experimental Example 3).

### Best Mode for carrying out the Invention

As described in the section BackgroundArt, CREBBP/EP300 inhibitory protein 1 (CRI1) of the present invention is one reported in each of Non-patent Documents 6 and 7 (GenBank Accession No. NM_014335). However, a CRI1 gene-derived promoter of the present invention has a sequence selected from the base sequence represented by chromosome 15 q21 (GenBank Accession No. NW_925884.1).

To be specific, the promoter forms the upstream portion of the CRI1 gene in the base sequence represented by GenBank Accession No. NW_925884.1 in Sequence Listing. When the transcriptional start site of the CRI1 gene is defined as +1, the promoter has a sequence selected from the region of -2586 to +84 (SEQ ID NO: 1), or more specifically selected from -1849 to +84 (SEQ ID NO: 2), selected from -1674 to +84 (SEQ ID NO: 3), selected from -1587 to +84 (SEQ ID NO: 4), selected from -1083 to +84 (SEQ ID NO: 5), selected from -766 to +84 (SEQ ID NO: 6), selected from -567 to +84 (SEQ ID NO: 7), selected from -366 to +84 (SEQ ID NO: 8), or selected from -296 to +84 (SEQ ID NO: 9), and is most preferably a promoter formed of the base sequence represented by -138 to +84 (SEQ ID NO: 10). Further, the promoter may be a promoter formed of the base sequence represented by -74 to +84 (SEQ ID NO: 11). The promoter formed of the sequence represented by SEQ ID NO: 1 in Sequence Listing can be represented by CRI1^{-2586/+84}, the promoter formed of the sequence represented by SEQ ID NO: 2 in Sequence Listing can be represented by CRI1^{-1849/+84}, the promoter formed of the sequence represented by SEQ ID NO: 3 in Sequence Listing can be represented by CRI1^{-1674/+84}, the promoter formed of the sequence represented by SEQ ID NO: 4 in Sequence Listing can be represented by CRI1^{-1587/+84}, the promoter formed of the sequence represented by SEQ ID NO: 5 in Sequence Listing can be represented by CRI1^{-1083/+84}_{,} the promoter formed of the sequence represented by SEQ ID NO: 6 in Sequence Listing can be represented by CRI1^{-766/+84}, the promoter formed of the sequence represented by SEQ ID NO: 7 in Sequence Listing can be represented by CRI1^{-567/+84}, the promoter formed of the sequence represented by SEQ ID NO: 8 in Sequence Listing can be represented by CRI1^{-366/+84}, the promoter formed of the sequence represented by SEQ ID NO: 9 in Sequence Listing can be represented by CRI1^{-296/+84}, the promoter formed of the sequence represented by SEQ ID NO: 10 in Sequence Listing can be represented by CRI1^{-138/+84}, and the promoter formed of the sequence represented by SEQ ID NO: 11 in Sequence Listing can be represented by CRI1^{-74/+84}.

A novel promoter of the present invention shows transcriptional activity in a mesothelioma-specific manner. The promoter of the present invention has significant transcriptional activity in malignant pleural mesothelioma cell lines such as 211H cells and H2452 cells, while the promoter has very little transcriptional activity in lung cancer-derived cell lines such as A549 cells derived from human squamous lung cancer and H322 cells derived from human bronchioloalveolar carcinoma, or has clearly low transcriptional activity as compared to that in mesothelioma-derived cell lines. Further, the promoter of the present invention has very little transcriptional activity in NHLF cells derived from normal human lung fibroblasts and normal mesothelial cells, or has clearly low transcriptional activity as compared to that in mesothelioma-derived cell lines.

A vector carrying the novel promoter of the present invention may be appropriately selected depending on the purposes of use, and a virus vector is suitably used. Further, an adenovirus (Ad) vector is suitably used as the virus vector. Ad which may be used in the present invention may be any as long as the Ad can in vivo or in vitro function as a vehicle for introducing sequences of nucleic acids such as DNA and RNA into a variety of types of cells, and is not particularly limited. Representative examples of the Ad include human Ad type 2, Ad type 5, Ad type 11, and Ad type 35 to be introduced into human host cells, and simian Ad, chimpanzee Ad, murine Ad, canine Ad, ovine Ad, and avian Ad to be introduced into non-human host cells. The Ad may be Ad that replicates only in particular cells, for example, E1-deleted Ad or conditionally replication-competent Ad. The E1-deleted Ad can proliferate only in 293 cells (having E1 in the cells), and the conditionally replication-competent Ad can replicate only in, for example, particular cancer cells.

The promoter of the present inventionmaybe incorporated into and carried by a vector capable of expressing the promoter together with a gene that should be expressed in a mesothelioma-specific manner. For example, a base sequence represented by any one of SEQ ID NOS: 1 to 11 may be selected as the base sequence of the promoter. The number of the promoters to be incorporated may be any as long as the length is such that the promoters can be incorporated into the vector, is not particularly limited, and a plurality of promoters may be incorporated. Further, the gene that can be carried by the vector and should be expressed in a mesothelioma-specific manner is referred to as a transgene in the present invention.

An example of the transgene in the present invention suitably includes a gene that damages mesothelioma cells, such as a cell death-inducing gene or a cell lysis-inducing gene. An example of the cell death-inducing gene includes a pro-apoptosis-related gene. As anti-apoptotic substances, there are given Bcl-2 and Bcl-XL as Bcl-2 family proteins each partially blocking the release of cy-tochrome c from mitochondria to inhibit apoptosis. In contrast, Bad binds to an anti-apoptotic protein out of the family proteins to inactivate the protein, to thereby activate procaspase and promote apoptosis. Further, Bax and Bak are stimulating factors for promoting the release of cytochrome c from mitochondria, and promote apoptosis. Bax and Bak are activated by pro-apoptotic Bcl-2 family members such as Bid. Accordingly, a specific example of the cell death-inducing gene includes a Bid gene. For example, the cell death-inducing gene or the cell lysis-inducing gene can be incorporated into the vector together with the promoter of the present invention to induce cell death or cell lysis in a mesothelioma-specific manner, and can be effectively used for treatment of mesothelioma without any influence on normal cells.

Further, when the vector is E1-deleted Ad, a gene encoding an E1 region can be used as the transgene and introduced into the vector together with the promoter of the present invention. This allows Ad to replicate in a mesothelioma-specific manner, leading to the disappearance of mesothelioma cells through the Ad infection of mesothelioma cells.

The production of the vector of the present invention can involve, in a production step, digesting each of one or more restriction enzyme-recognizing sequences with a restriction enzyme, and introducing a transgene by in vitro ligation via a shuttle vector or not via the shuttle vector.

The vector, e.g., Ad vector of the present invention may be produced by a production method including the following steps :
1) constructing an expression construct including the promoter sequence of the present invention in an untranslated region of a transgene;
2) constructing a shuttle vector including the expression construct in the step 1);
3) preparing an Ad genome; and
4) cleaving the Ad genome with a restriction enzyme, and ligating the gene-expressing shuttle vector produced in the step 2) to the cleaved Ad genome.

The present invention also encompasses a vector containing a promoter, and more specifically, a recombinant vector carrying the promoter and a transgene downstream of the promoter. A specific example of the transgene suitably includes a gene that damages mesothelioma cells, such as a cell death-inducing gene or a cell lysis-inducing gene. An example of the cell death-inducing gene includes a pro-apoptosis-related gene. For anti-apoptotic substances, Bcl-2 family proteins such as Bcl-2 and Bcl-XL partially block the release of cytochrome c from mitochondria to inhibit apoptosis. In contrast, Bad binds to an anti-apoptotic protein out of the family proteins to inactivate the protein, to thereby activate procaspase and promote apoptosis. Further, Bax and Bak are each a stimulating factor promoting the release of cytochrome c from mitochondria, and promote apoptosis. Bax and Bak are activated by pro-apoptotic Bcl-2 family members such as Bid. Accordingly, a specific example of the cell death-inducing gene includes a Bid gene.

A vector carrying the above-mentioned transgene downstream of the promoter of the present invention can be utilized in a therapeutic agent for mesothelioma. The present invention also encompasses a therapeutic agent for mesothelioma including, as an active ingredient, a recombinant vector containing the transgene.

In addition, the vector containing the promoter of the present invention may also be used in the inspection of mesothelioma. To be specific, a virus vector carrying a marker gene downstream of the promoter is used. Because the promoter of the present invention has significant transcriptional activity in mesothelioma, a marker gene is expressed on the basis of the presence of mesothelioma, which allows for the inspection of mesothelioma. The marker gene may be any as long as the gene can express a protein capable of being used for the inspection. Examples of the marker gene include, but are not particularly limited to, a fluorescent protein, and more specifically, a Green Fluorescent Protein (GFP). The present invention also encompasses a virus vector for inspection of mesothelioma, which can express a marker gene on the basis of the presence of mesothelioma, and a method for inspection of mesothelioma, including observing the presence or absence of the expression of a marker by using the virus vector for inspection of mesothelioma.

### Examples

Hereinafter, as for the promoter of the present invention and the recombinant vector containing the promoter, the present invention is described in more detail by way of examples. It is apparent that the present invention is not limited to these examples.

### (Example 1) Confirmation of transcriptional activity of various promoters in various cells

### 1) Construction of expression constructs including various mesothelioma marker gene-derived promoters

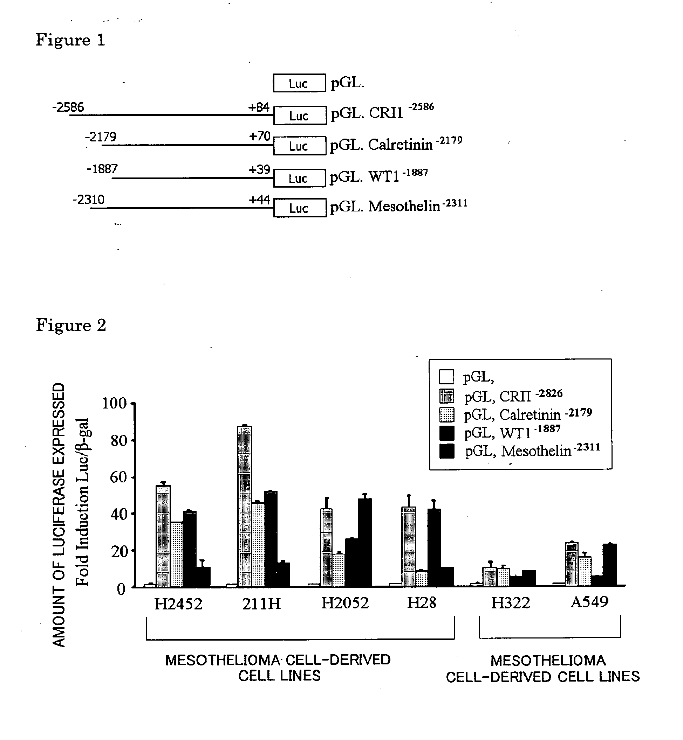

As for CRI1, calretinin, Wilms' tumor susceptibility gene 1 (WT1), and mesothelin as mesothelioma markers, expression constructs were constructed by excising promoter regions from the respective marker genes, and allowing the regions to bind to firefly luciferase genes. FIG. 1 is a schematic diagram illustrating expression constructs including the respective promoters. Here, a calretinin gene-derived promoter has a sequence selected from the sequence of chromosome 16 q21.1 (GenBank Accession No. NT_010498.15), a WT1 gene-derived promoter has a sequence selected from the sequence of chromosome 11 p3 (GenBank Accession No. NT_079237.17), and a mesothelin gene-derived promoter has a sequence selected from the sequence of chromosome 16 (GenBank AccessionNo. NT_037887.4). When the transcriptional start site of each of the marker genes is defined as +1, the sequence of each of the promoter regions is represented by any one of SEQ ID NOS: 2 or 12 to 14 in Sequence Listing, and is specifically as follows:
CRI1 gene promoter: -2586/+84 (SEQ ID NO: 2);
Calretinin gene promoter: -2179/+70 (SEQ ID NO: 12);
WT1 gene promoter: -1887/+39 (SEQ ID NO: 13); and
Mesothelin gene promoter: -2310/+44 (SEQ ID NO: 14).

### 2) Confirmation of transcriptional activity of respective promoters in mesothelioma or lung cancer cells

Expression constructs were produced by inserting the respective promoters described above into pGL3 luciferase reporter vectors (Promega) (pGL3 Luciferase Reporter Vectors, Promega, see Technical Manual No. 033) Cells derived from four kinds of malignant pleural mesothelioma cell lines (H2452, 211H, H2052, and H28) and two kinds of lung cancer cell lines (A549 and H322) were each seeded in triplicate into a 6-well plate at a cell count of about 4×10⁶, and the cells were each confirmed for their survival. After that, a transfection reagent Lipofectin (registered trademark) (Invitrogen) was used to transfect the cells with 2 µg each of the expression constructs. After 24 hours, luciferase light emission was measured by a luciferase assay in each of the cells to confirm the transcriptional activity of each of the promoters.

As a result, there was a tendency that each of the marker gene-derived promoters shows transcriptional activity in a mesothelioma cell-specific manner and shows low transcriptional activity in lung cancer cells. In particular, the CRI1 gene promoter: -2586/+84 had transcriptional activity in a mesothelioma cell-specific manner and had only low transcriptional activity in lung cancer cells, as compared to other promoters (FIG. 2).

### 3) Confirmation of transcriptional activity of various mesothelioma marker gene-derived promoters in normal cells

With the use of the same technique as that in the item 2), the respective expression constructs were produced by inserting the respective promoter regions into pGL3 luciferase reporter vectors (Promega). Normal mesothelial cells, normal pleural cells (4/4RM-4 cells derived from rat pleura), and NHLF cells derived from normal human lung fibroblasts were each cultured in the same manner as in the item 2), and the cells were each confirmed for their survival. After that, the cells were each transfected with 2 µg each of the expression constructs. After 24 hours, luciferase light emission was measured by a luciferase assay in each of the cells to confirm the transcriptional activity of each of the promoters.

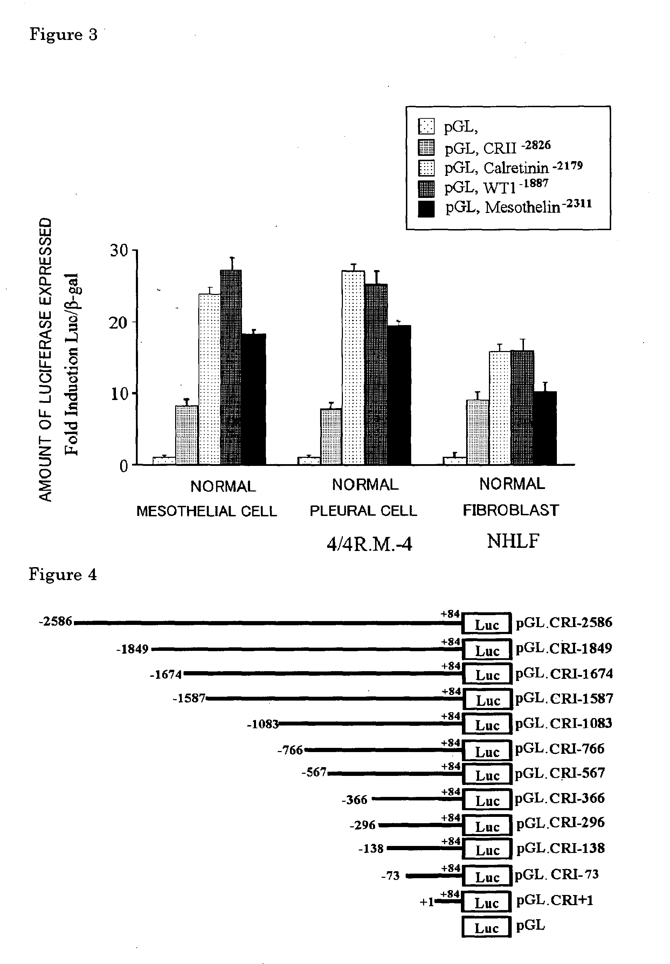

As a result, the CRI1 gene promoter: -2586/+84 (CRI1^{-2586/+84}) had low transcriptional activity in normal cells, while each of other marker-derived promoters had transcriptional activity in normal cells as well (FIG. 3).

Those results confirmed that CRI1^{-2586/+84} had low transcriptional activity in normal cells and lung cancer cells and had high transcriptional activity in mesothelioma cells, and thus exerted transcriptional activity in a mesothelioma-specific manner.

### (Example 2) Confirmation of transcriptional activity of CRI1 gene-derived promoter in various cells

### 1) Construction of expression construct including CRI1 gene-derived promoter

As for promoter regions of a CRI1 gene, expression constructs were constructed by excising the respective regions having different lengths, and allowing the regions to bind to firefly luciferase genes. FIG. 4 is a schematic diagram illustrating expression constructs including the respective promoters.

When the transcriptional start site of the CRI1 gene is defined as +1, the respective promoters are formed of base sequences represented by the following SEQ ID NOS:
CRI1^{-2586/+84} (SEQ ID NO: 1);
CRI1^{-1849/+84} (SEQ ID NO: 2);
CRI1^{-1674/+84} (SEQ ID NO: 3);
CRI1^{-1587/+84} (SEQ ID NO: 4);
CRI1^{-1083/+84} (SEQ ID NO : 5);
CRI1^{-766/+84} (SEQ ID NO: 6);
CRI1^{-567/+84} (SEQ ID NO: 7);
CRI1^{-366/+84} (SEQ ID NO: 8);
CRI1^{-296/+84} (SEQ ID NO: 9);
CRI1^{-138/+84} (SEQ ID NO: 10); and
CRI1^{-74/+84} (SEQ ID NO: 11).

### 2) Confirmation of transcriptional activity of respective promoters in respective cells

With the use of the same technique as that in Example 1 above, the respective expression constructs were produced by inserting the respective promoters derived from the CRI1 gene into pGL3 luciferase reporter vectors (Promega). Two kinds of malignant pleural mesothelioma cell lines (H2452 and MSTO-211H), two kinds of lung cancer cell lines (A549 and H322), and two kinds of normal cell lines (normal mesothelial cells and NHLF) were cultured in the same manner as in Example 1, and the cells were each confirmed for their survival. After that, the cells were each transfected with 2 µg each of the expression constructs. After 24 hours, luciferase light emission was measured by a luciferase assay in each of the cells to confirm the transcriptional activity of each of the promoters.

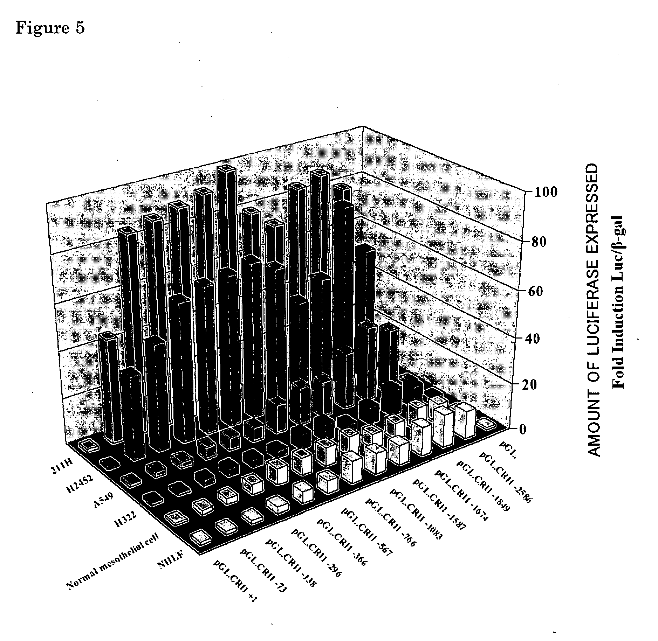

As a result, all of the respective CRI1 gene-derived promoters had strong transcriptional activity in malignant pleural mesothelioma cells and showed low transcriptional activity in lung cancer cells and normal cells. In particular, in the case of using each of the promoters CRI1^{-296/+84}, CRI1^{-138/+84}, and CRI1^{-74/+84}, higher mesothelioma specificity was observed, and more particularly, in the case of using CRI1^{-138/+84}, highest mesothelioma specificity was observed (FIG. 5).

### (Example 3) Production of therapeutic, genetically-modified adenovirus (Ad) vector

An Ad vector carrying a transgene and a CRI1 gene promoter (CRI1^{-138/+84}) upstream of the transgene was produced. A cell death-inducing gene (BID) or an Ad early gene E1 was used as the transgene.

### 1) Construction of expression construct including promoter sequence of present invention in untranslated region of transgene

The cell death-inducing gene (BID) and hemagglutinin (HA) gene sequences bound to each other (A) or the Ad early gene E1 (B) was used as the transgene. Each of the expression constructs was produced by allowing four tandem repeats of CRI1^{-138/+84} to bind to an upstream region of (A) or (B).

### 2) Construction of shuttle vectors including expression constructs in above item 1)

Shuttle vectors including the expression constructs constructed in the item 1) were constructed in accordance with the method described in Tong-Chuan He et al., Proc. Natl. Acad. Sci. USA, 95: 2509-2514, 1998.

### 3) Production of Ad vector

An E1-deleted type 5 Ad genome was prepared, the Ad genome was cleaved with a restriction enzyme, and the gene-expressing shuttle vectors produced in the item 2) were subjected to homologous recombination in accordance with the method of He et al., to thereby afford Ads carrying various expression constructs described above (Ad-CR1^{-138 4x} /HA-BID and Ad-CRI1^{-138 4x} /E1A). In this example, in order to distinguish a BID expression construct from an intrinsic BID, the construct was allowed to bind to an HA tag.

### (Experimental Example 1) Effect of Ad-CRI1^{-138 4x}/HA-BID on mesothelioma cells

Examination was made on a cell-killing effect of Ad-CRI1^{-138 4x}/HA-BID obtained in Example 3 on mesothelioma cells. Ad-CRI1^{-138 4x}/GFP containing a green fluorescent protein (GFP) -expressing gene produced by the same technique was used as a control. Here, the Ad vectors are both E1-deleted vectors and are replication-incompetent in mesothelioma cells, but differ in that one includes a cell death-inducing gene (BID) and the other includes a non-toxic GFP gene.

Each of the resultant Ad vectors was infected to two kinds of malignant pleural mesothelioma cell lines (H2452 and 211H), two kinds of lung cancer cell lines (H322 and A549), two kinds of normal cell lines (normal mesothelial cells and NHLF), and two kinds of cancer cell lines other than lung cancer cell lines (liver cancer cells: Hep3B and breast cancer cells: MCF7). The cell death in each of the cells after Ad infection was quantified by flow cytometry after propidium iodide (PI) staining.

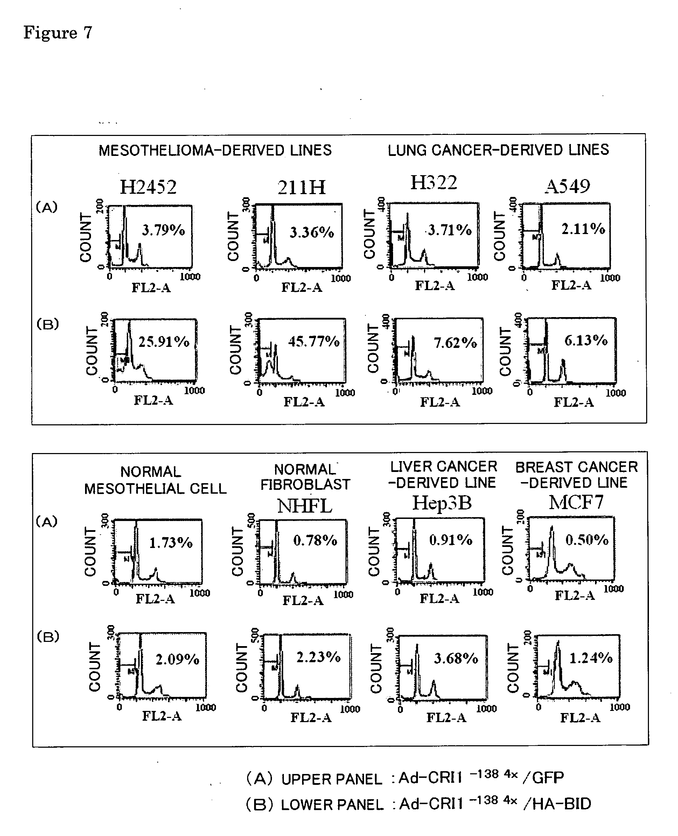

As a result, as illustrated in FIGS. 7, in the two kinds of malignant pleural mesothelioma cell lines (H2452 and 211H) infected with the Ad including the cell death-inducing gene, an increase in sub-G₀/G₁ population having a peak between G₁ and G₀ in the cell cycle was observed, and hence, the occurrence of apoptosis was confirmed. Meanwhile, in the lung cancer cells, normal cells, and other cancer cells, there was no difference in flow cytometric patterns between cases with and without the cell death-inducing gene. Those results confirmed that Ad-CRI1^{-138 4x}/HA-BID was expressed in a mesothelioma cell-specific manner.

### (Experimental Example 2) Effect cf Ad-CRI1^{-138 4x}/E1A on mesothelioma cells

Examination was made on an effect of Ad-CRI1^{-138 4x}/E1A obtained in Example 3 on mesothelioma cells. In the same manner as in Experimental Example 1, Ad-CRI1^{-138 4x}/GFP was used as a control. Here, there is a difference in that the Ad vector including the Ad early gene E1 is replication-competent in mesothelioma cells and the Ad vector including the GFP gene is replication-incompetent in mesothelioma cells.

Each of the resultant Ad vectors was introduced into two kinds of malignant pleural mesothelioma cell lines (H2452 and MSTO-211H) and two kinds of normal cell lines (normal mesothelial cells and NHLF) to measure the number of viable cells. The number of the viable cells was measured by an MTS assay (assay involving measuring at 490 nm water-soluble formazan released into a culture medium on the basis of a conversion reaction of a tetrazolium salt (MTS*[3-(4,5-dimethylthiazol-2-yl)-5-(3carboxymethoxyphenyl)-2-(4-sulphophenyl)-2H-tetrazolium, inner salt]) to formazan in viable cells).

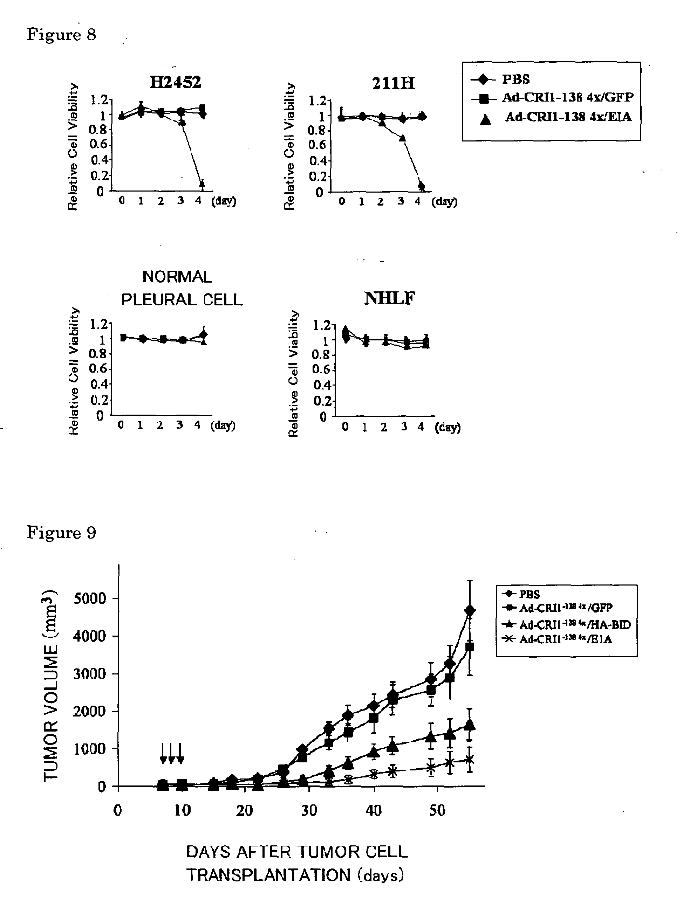

As a result, as illustrated in FIGS. 8, it was confirmed that the infection of AD-CRI1^{-138 4x}/E1A clearly decreased mesothelioma cells as compared to the cases of the infection of Ad-CRI1^{-138 4x}/GFP and only PBS and provided no difference from the cases of the infection of Ad-CRI1^{-138 4x}/GFP and PBS in normal cells. Those results confirmed that Ad-CRI1^{-138 4x}/E1A also had a cell-killing effect in a mesothelioma cell-specific manner.

### (Experimental Example 3) Effect of Ad-CRI1^{-138 4x}/HA-BID or Ad-CRI1^{-138 4x}/E1A on mesothelioma (in vivo)

Examination was made on an in vivo cell-killing effect of Ad-CRI1^{-138 4x}/HA-BID or AD-CRI1^{-138 4x}/E1A obtained in Example 3 on mesothelioma cells. In the same manner as in Experimental Examples 1 and 2, Ad-CRI1^{-138 4x}/GFP containing the GFP-expressing gene was used as a control vector. Here, the Ad vector including the cell death-inducing gene (BID) or the non-toxic GFP gene is an E1-deleted vector and is replication-incompetent in mesothelioma cells. Further, the Ad vector including the Ad early gene E1 is replication-competent in mesothelioma cells.

A mouse model of mesothelioma was produced by subcutaneously inoculating 2.5×10⁶ 211H cells to 6-week-old female BALB/c nude mice. To the mouse model of mesothelioma on day 8 after the inoculation of 211H cells, PBS, Ad-CRI1^{-138 4x} /GFP, Ad-CRI1^{-138 4x}/HA-BID, or Ad-CRI1^{-138 4x}/E1A was locally administered at 5×10⁷ plaque forming units (pfu) for 3 consecutive days, and the size of a tumor was observed for 56 days after the inoculation (n=8 for each of the conditions).

As a result, as illustrated in FIG. 9, the infection of Ad-CRI1^{-138 4x}/HA-BID or Ad-CRI1^{-138 4x}/E1A provided an anti-tumor effect as compared to the case of the infection of Ad-CRI1^{-138 4x}/GFP or PBS alone as a control.

### Industrial Applicability

As mentioned in detail above, it was confirmed that the novel promoter of the present invention had transcriptional activity in a mesothelioma-specific manner. It was also confirmed that the introduction of a vector carrying the novel promoter, a cell death-inducing gene, and the like into cells provided an apoptosis action in a mesothelioma-specific manner and showed a cell-killing action. Further, an anti-tumor effect was confirmed in vivo as well. From those results, the vector including the novel promoter of the present invention may be utilized in the case where a certain mesothelioma-specific action is required. For example, the introduction of the vector into cells together with the cell death-inducing gene or cell lysis-inducing gene as mentioned above can damage cells in a mesothelioma-specific manner. Those results suggest that the vector including the novel promoter of the present invention can serve as an effective therapeutic agent for mesothelioma. In addition, the vector including the novel promoter and the marker gene of the present invention can also be used in the inspection of mesothelioma.

## Claims

1. A novel promoter, comprising a CREBBP/EP300 inhibitory protein 1 (CRI1) gene-derived promoter, wherein the promoter shows transcriptional activity in a mesothelioma-specific manner.

2. A novel promoter according to claim 1, wherein the CRI1 gene-derived promoter has a sequence selected from a region of -2586 to +84 in a CRI1 gene.

3. A novel promoter according to claim 1 or 2, wherein the CRI1 gene-derived promoter has a sequence represented by any one of SEQ ID NOS: 1 to 11 in Sequence Listing.

4. A virus vector, comprising the novel promoter according to any one of claims 1 to 3.

5. A virus vector according to claim 4, wherein the virus vector comprises an adenovirus vector.

6. A virus vector according to claim 5, wherein the adenovirus comprises a conditionally replication-competent adenovirus.

7. A virus vector according to any one of claims 4 to 6, further comprising a cell death-inducing gene and/or a cell lysis-inducing gene downstream of the promoter.

8. A gene therapy vector for treatment of mesothelioma, comprising the virus vector according to any one of claims 4 to 7.

9. A therapeutic agent for mesothelioma, comprising the gene therapy vector for treatment of mesothelioma according to claim 8.

10. A virus vector according to any one of claims 4 to 6, further comprising a marker gene downstream of the promoter.

11. A virus vector according to claim 10, wherein the marker gene comprises a fluorescent protein-expressing gene.

12. A virus vector for inspection of mesothelioma, comprising the virus vector according to claim 10 or 11.

13. A method for inspection of mesothelioma, comprising observing a presence or absence of expression of a marker by using the virus vector for inspection of mesothelioma according to claim 12.
